# EUROPÄISCHE PATENTSCHRIFT

(11) **EP 2 007 348 B1**
(45) Veröffentlichungstag und Bekanntmachung des Hinweises auf die Patenterteilung: **10.12.2014**
(21) Anmeldenummer: 07711788.5
(22) Anmeldetag: 05.03.2007
(51) Int. Cl.: A61K 9/00, A23L 1/29, A23L 1/305, A61K 31/198, A61K 38/05, A61P 1/00, A61P 13/12

(54) **PAEDIATRISCHE AMINOSÄURELÖSUNG ZUR PARENTERALEN ERNÄHRUNG**
PAEDIATRIC AMINO ACID SOLUTION FOR PARENTERAL NUTRITION
SOLUTION D'ACIDES AMINÉS POUR LA NUTRITION PARENTÉRALE PÉDIATRIQUE

(30) Priorität: 20.04.2006 DE 102006018293
(43) Veröffentlichungstag der Anmeldung: 31.12.2008
(73) Patentinhaber: Fresenius Kabi Deutschland GmbH, 61352 Bad Homburg v.d.H. (DE)
(72) Erfinder: BRAND, Ortrud, 73760 Ostfildern (DE); ERBE, Thorsten, 35510 Butzbach (DE); ACHLEITNER, Georg, 8010 Graz (AT); FEICHTINGER, Norbert, 8010 Graz (AT)
(74) Vertreter: Fresenius Kabi Deutschland GmbH
(86) Internationale Anmeldenummer: PCT/EP2007/001878
(87) Internationale Veröffentlichungsnummer: WO 2007/121807

(56) Entgegenhaltungen:
- EP-A1- 0 148 680
- EP-A2- 0 087 750
- WO-A-91/16067
- WO-A-92/09277
- WO-A-2005/030242
- US-A- 5 432 160
- US-A- 5 561 111
- LACEY JM ET AL: "The effects of glutamine-supplemented parenteral nutrition in premature infants" JPEN, Bd. 20, Nr. 1, 1996, Seiten 74-80, XP009083795

## Beschreibung

Die Erfindung betrifft eine Aminosäurelösung zur parenteralen Ernährung pädiatrischer Patienten enthaltend Aminosäuren nach dem Oberbegriff des Anspruchs 1. Die Lösung eignet sich insbesondere zur parenteralen Ernährung von Früh- und Neugeborenen, Säuglingen und Kleinkindern, entweder als parenterales Supplement oder in Kombination mit anderen Nährlösungen als totale parenterale Ernährung (TPN). Die Erfindung betrifft weiterhin ein Verfahren zur Herstellung von genannten Aminosäurelösungen.

Wenn die vollständige Ernährung über den oralen/enteralen Weg nicht sichergestellt werden kann, ist eine vollständige oder zusätzliche parenterale Ernährungstherapie indiziert. Die Aufnahme der Nahrung erfolgt in diesem Fall auf intravenösem Weg. Dies ist insbesondere bei einer Obstruktion des Magen-/Darmtraktes notwendig. Häufig treten bei Früh- und Neugeborenen, Säuglingen und Kleinkindern Missbildungen oder Entzündungen des Darms auf. Beispiele hierfür sind entzündliche Darmerkrankungen wie Morbus Crohn, Colitis ulcerosa etc. sowie gastrointestinale Fisteln, maligne Erkrankungen des Darms, Kurzdarm-Syndrom oder mangelnde Ausbildung des Darms zum Zeitpunkt der Geburt. Weiterhin kann parenterale Ernährung im perioperativen Bereich, bei Intensivpatienten, bei Sepsis oder therapieresistenter Diarrhöe indiziert sein.

Eine geeignete Aminosäurelösung für Säuglinge und Kleinkinder unterscheidet sich in den Anforderungen an das Aminosäuremuster stark von Aminosäurelösungen für Erwachsene. Bezogen auf das Körpergewicht benötigen Kleinkinder deutlich mehr Aminosäuren. Der Metabolismus eines Kleinkindes unterscheidet sich ebenfalls von dem eines Erwachsenen. Verschiedene Aminosäuren, die beim Erwachsenen als nicht-essentiell gelten, müssen beim Früh- und Neugeborenen sowie beim Kleinkind als essentiell angesehen werden, weil der Körper diese Aminosäuren erst ab einem gewissen Alter selbst in ausreichenden Mengen synthetisieren kann. Andere Aminosäuren können zwar auch bereits vom Kleinkind synthetisiert werden, dies geschieht allerdings in einer geringeren Geschwindigkeit als dies beim Erwachsenen der Fall ist. Daher kann es bei der Verwendung einer für Erwachsene konzipierten

Aminosäurelösung bei der Anwendung bei Früh- und Neugeborenen sowie Kleinkindern zu Mangel- und / oder Überschusserscheinungen für bestimmte Aminosäuren kommen. Dies ist vor allem für die Aminosäuren Glutamin, Tyrosin, Cystein, Taurin, Methionin und Phenylalanin der Fall.

Aufgrund des insbesondere bei Früh- und Neugeborenen noch nicht voll ausgebildeten Aminosäuren-Stoffwechsels ist die Ausgewogenheit der Mischung von besonderer Wichtigkeit, da etwaige Unregelmäßigkeiten vom jungen Organismus schwerer auszugleichen sind. Einer präzisen und kontinuierlichen parenteralen Ernährung muss bei Früh- und Neugeborenen und beim Kleinkind im Bedarfsfall weiterhin besondere Bedeutung beigemessen werden, weil der Körper noch keine ausgeprägten eigenen Reserven angelegt hat, durch die mögliche Mangelerscheinungen abgemildert werden könnten. Erst in einem Alter von > 2 Jahren fängt der Stoffwechsel von Kindern an sich dem von Erwachsenen anzunähern. Obwohl die erfindungsgemäße Aminosäurelösung speziell auf die Bedürfnisse von pädiatrischen Patienten abgestimmt ist, ist eine Verwendung auch für Erwachsene denkbar, bei denen es aufgrund pathophysiologischer Stoffwechselveränderungen zu ähnlichen Mangelerscheinungen kommen kann. Die ist vor allem für Patienten mit Nieren- oder Lebererkrankungen der Fall.

Es ist daher unabdinglich, unter Beachtung verschiedenster Kriterien, wie später im Detail geschildert, das Aminosäuremuster für eine Aminosäurelösung zur parenteralen Ernährung von Früh- und Neugeborenen sowie Kleinkindern neu zu definieren. Dabei ist nicht nur die Menge der einzelnen Aminosäuren entscheidend, sondern auch die Art der Verabreichung sowie eine gute technologische Umsetzung bei der Herstellung der Lösung; dies gilt in besonderem Maße für die Sterilisationsbedingungen.

EP 0 148 680 A1 beschreibt eine den pädiatrischen Bedürfnissen angepasste Aminosäurelösung zur parenteralen Ernährung. Die Aminosäuren Cystein, Taurin und Tyrosin sind als semi-essentiell gekennzeichnet.

DE 2531201 A1 zeigt auf, wie bei parenteralen pädiatrischen Aminosäurelösungen ein ausgeglichener Stickstoffhaushalt erhalten werden kann. Es werden außerdem die Vorteile der Verabreichung von abgestimmten Mengen von freien Aminosäuren im Vergleich zur Verabreichung von Proteinhydrolysaten diskutiert.

WO91/16067 A1 beschreibt den Einsatz von Oligopeptiden für eine für pädiatrische Zwecke einsetzbare parenterale Ernährungslösung.

Der allgemeine Einsatz von Oligopeptiden, insbesondere Tri- und Dipeptiden, für Aminosäurelösungen zur parenteralen Ernährung und die damit verbundenen positiven Effekte für Löslichkeit und Produktstabilität werden in US 5 432 160, DE 31 08 079 C2, EP 0 087 751 A1 and EP 0 087 750 B1 diskutiert.

LACEY JM ET AL: "The effects of glutamine-supplemented parenteral nutrition in premature infants", JPEN, Bd. 20, Nr. 1, 1996, Seiten 74-80, XP009083795 offenbart, dass Glutamin in der Entwicklung von Frühgeborenen eine wesentliche Rolle spielt. Durch die Ergänzung von Glutamin bei der parenteralen Ernährung von Frühgeborenen kann beispielsweise der Aufenthalt im Krankenhaus verkürzt und die enterale Ernährung gefördert werden. Neben Glutamin kann die parenterale Ernährungslösung weitere Aminosäuren in unterschiedlichen Konzentrationen enthalten.

EP 0 087 750 A2 offenbart, dass die wasserlöslichen α-aminoacylierten Derivate des Glutamins sich als Glutaminquelle in oralen Aminosäure-Präparaten und in Infusionslösungen zu parenteralen Ernährung von Patienten eignen, da sie im Organismus allmählich aufgespalten werden, ohne dass die Bildung von toxischen Cyclisierungsprodukten des Glutamins zu befürchten ist.

US 5 432 160 offenbart eine ß-Alanyl-L-Glutamin enthaltende Nährstoffzusammensetzung, welche eine gute Wasserlöslichkeit und eine hohe Wärmestabilität aufweist. Diese Nährstoffzusammensetzung kann ebenfalls verschiedene Aminosäuren enthalten.

Aus keiner der vorgenannten Schriften geht hervor, dass es unter Verwendung von Oligopeptiden zur Bereitstellung von Aminosäuren möglich ist, Mengen an bestimmten Aminosäuren zu verabreichen, die auf einem anderen als dem bisher beschriebenen Niveau liegen, aber für die Entwicklung des neonatalen Organismus besser angepasst sind.

Der Erfindung liegt daher die Aufgabe zugrunde, eine Aminosäurelösung zur parenteralen Ernährung bereitzustellen, die ein speziell an die Bedürfnisse von Früh- und Neugeborenen, Säuglingen und Kleinkindern angepasstes Aminosäuremuster aufweist, das alle benötigten Aminosäuren in ausreichender Menge und Relation anbietet. Ein besonderes Augenmerk liegt dabei auf der Bereitstellung von adäquaten Mengen der Aminosäuren Glutamin, Tyrosin, Cystein und Taurin in leicht metabolisierbarer Form sowie der damit verbundenen Reduzierung der Aminosäuren Glutaminsäure, Phenylalanin und Methionin.

Die Aufgabe wird durch Bereitstellen einer Aminosäurelösung zur parenteralen Ernährung pädiatrischer Patienten gemäß Anspruch 1 und der Verwendung in Anspruch 7 gelöst.

Eine weitere Aufgabe der Erfindung betrifft die Bereitstellung einer vollständigen parenteralen Ernährung, die alle benötigten Aminosäuren in ausreichender Menge und Relation anbietet.

Die Aufgabe wird durch Bereitstellen einer medizinischen Zusammensetzung gemäß Anspruch 10 und der Verwendung in Anspruch 12 gelöst.

Eine weitere Aufgabe, die die Optimierung des Sterilisationsverfahrens einer erfindungsgemäßen Lösung betrifft, wird durch Anspruch 13 gelöst.

Vorteilhafte Ausführungsformen werden in den Unteransprüchen aufgezeigt.

In Abhängigkeit von Körpergewicht und Entwicklungszustand des Früh- und Neugeborenen soll bei der parenteralen Ernährung ein Volumen von 100 - 160 ml pro kg Körpergewicht (KG) und Tag nicht überschritten werden. Um auf der einen Seite bei der Verabreichung lokale Überkonzentrationen zu vermeiden, auf der anderen Seite aber auch die Mischbarkeit mit anderen Komponenten, wie Lösungen von Kohlehydraten, Fetten, Elektrolyten, Vitaminen und Spurenelementen, zu gewährleisten, ohne dass auf diese Weise eine Überschreitung der Volumengrenze stattfindet, enthält die Aminosäurelösung 3-30 % w/w Proteinbausteine, bevorzugt 5- 20 % w/w Proteinbausteine und besonders bevorzugt 7-15 % w/w Proteinbausteine. Die Proteinbausteine liegen bevorzugt in Form von freien Aminosäuren, Di- oder Tripeptiden vor, der Gehalt von Peptiden mit einer Kettenlänge von > 5 oder Proteinen ist kleiner als 5 % der Proteinbausteine, bevorzugt kleiner als 1 %.

Die Aminosäurelösung zur parenteralen Ernährung von Früh- und Neugeborenen, Säuglingen und Kleinkindern unterscheidet sich von üblichen Aminosäurelösungen zur parenteralen Ernährung von Erwachsenen in Mengengehalt und dem Aminosäuremuster.

Im Gegensatz zum Erwachsenen, bei dem sich bei konstantem Gewicht Synthese und Abbau von Proteinen etwa die Waage halten, muss bei Früh- und Neugeborenen sowie bei Kleinkindern aufgrund des rapiden Wachstums der Körpermasse die Synthese deutlich überwiegen. Aus diesem Grunde ist der Tagesbedarf an Aminosäuren bei diesen Patienten im Vergleich zu Erwachsenen bezogen auf das Körpergewicht deutlich erhöht. Die Tagesdosis der durch die Aminosäurelösung verabreichten Proteinbausteine ist 2-4 g, bevorzugt 2,5-3,5 g, pro Kilogramm Körpergewicht, um den gesamten Tagesbedarf des pädiatrischen Patienten zu decken. Für übliche Berechnungen wird eine Menge von 3 g Proteinbausteinen pro Kilogramm Körpergewicht genutzt.

Alle angegebenen Aminosäuren können in freier Form oder in Form ihrer Vorstufen zur Verfügung gestellt werden, wobei Glutamin in Form von Alanyl-Glutamin, Glycyl- Glutamin oder Mischungen davon vorliegt und Tyrosin in Form von Glycyl-Tyrosin, Alanyl-Tyrosin oder Mischungen davon vorliegt. Sollten die Aminosäuren in Form ihrer Vorstufen bereitgestellt werden, so beziehen sich die Mengenangaben für die Aminosäuren nur auf den Aminosäureanteil der Vorstufen. In allen Fällen werden bevorzugt linksdrehende Aminosäuren eingesetzt.

Neben einem erhöhten Tagesbedarf von Früh- und Neugeborenen sowie Kleinkindern unterscheidet sich deren Stoffwechsel aber in besonderer Weise von dem von Erwachsenen, indem verschiedene Aminosäuren im Vergleich mit verminderter Geschwindigkeit gebildet und / oder abgebaut werden. Einige Aminosäuren, die beim Erwachsenen endogen synthetisiert werden und somit als nicht-essentiell gelten, können vom Früh- und Neugeborenen sowie vom Kleinkind nicht ausreichend endogen gebildet werden und müssen dort daher als essentiell bzw. semi-essentiell gelten. Diese Aminosäuren, deren Zufuhr damit besondere Bedeutung zukommt, sind Cystein, Tyrosin, Glutamin und Taurin. Andere Aminosäuren werden von Früh- und Neugeborenen sowie Kleinkindern nur mit verringerter Rate abgebaut, dazu zählen Phenylalanin und Methionin.

Glutamin ist die am Häufigsten vorkommende Aminosäure. Sie tritt vermehrt im Plasma und in Muskeln auf. Glutamin ist als unverzichtbar besonders für Zellen mit hoher Mitose-Rate anzusehen. Dazu gehören z.B. Zellen wie Lymphozyten und Enterozyten. Eine ausreichende Versorgung mit Glutamin gewährleistet somit u.a. auch ein funktionierendes Immunsystem. Weiterhin ist Glutamin für Aufbau und Erhalt der Muskelmasse unabdingbar. Da der neonatale Organismus Glutamin nicht in ausreichender Menge selbst herstellen kann, ist eine angemessene Versorgung mit dieser Aminosäure im Rahmen einer parenteralen Ernährung besonders wichtig. Dies gilt weiterhin im Hinblick auf die Synthese von Purinen und Pyrimidinen. Glutamin stellt für diese Bausteine der DNA eine Vorstufe dar. Glutamin ist daher besonders wichtig in dem im Wachstum begriffenen Körper eines Früh- und Neugeborenen sowie des Kleinkindes, in dem vermehrt Zellteilung stattfindet.

Daneben ist Glutamin eine Vorstufe von Glutathion, dem eine bedeutende Rolle als Antioxidanz zukommt.

Aus diesen Gründen enthält die erfindungsgemäße parenterale Komposition einen erhöhten Anteil an Glutamin, der 9-30 g, bevorzugt 11 - 25 g und besonders bevorzugt 13-20 g Glutamin auf 100 g Aminosäuren (AA) beträgt. Als Tagesdosis werden pro Kilogramm Körpergewicht 0,25-0,90 g, bevorzugt 0,3 - 0,75 g und besonders bevorzugt 0,39 - 0,6 g Glutamin eingesetzt.

Eine weitere bei Erwachsenen als nicht-essentiell angesehene Aminosäure, Tyrosin, muss bei Früh- und Neugeborenen sowie Kleinkindern als eine, zumindest teilweise, essentielle Aminosäure erachtet werden. Der neonatale Organismus ist nicht oder nur sehr begrenzt in der Lage, Tyrosin aus Phenylalanin zu synthetisieren. Der Grund ist die mangelnde Expression des dafür verantwortlichen Enzyms Phenylalaninhydroxylase. Als Folge dessen ist gleichzeitig der Abbau von Phenylalanin im Vergleich zum erwachsenen Organismus verringert. Neben der allgemeinen Funktion als Baustein für den Aufbau von Proteinen ist Tyrosin ein Vorläufer für Norepinephrin, Dopamin, Thyroxin und weiterer Hormone. Damit ist die ausreichende Versorgung mit Tyrosin für einen geregelten Hormonhaushalt und für den Aufbau des Nervensystems von größter Wichtigkeit.

Aus diesen Gründen enthält die parenterale Komposition einen erhöhten Anteil an leicht metabolisierbarem Tyrosin, der 1-4 g, bevorzugt 1,2 - 3 g und besonders bevorzugt 1,5-2,5 g Tyrosin auf 100g Aminosäuren beträgt. Als Tagesdosis werden pro Kilogramm Körpergewicht 30-120 mg, bevorzugt 35 - 90 mg und besonders bevorzugt 45 - 75 mg Tyrosin eingesetzt.

Tyrosin und Phenylalanin konkurrieren auf ihren Transportwegen in das Gewebe miteinander. Ein erhöhter Phenylalaninspiegel hemmt somit die Aufnahme von Tyrosin. Wegen des verringerten Abbaus von Phenylalanin einerseits, und andererseits um die Gesamtmenge der aromatischen Aminosäuren in Relation zu den anderen Aminosäuren (AA) konstant zu halten, ist die Menge an Phenylalanin in der erfindungsgemäßen Aminosäurenlösung reduziert. Die Gesamtmenge aromatischer Aminosäuren sollte 7 g/100g AA, bevorzugt 6g/100g AA und besonders bevorzugt 5,6 g/100g AA, nicht überschreiten.

Die parenterale Komposition enthält 3-5 g, / 100g AA bevorzugt 3,3-4 g / 100g AA und besonders bevorzugt 3,5-3,8 g / 100g AA Phenylalanin. Das Verhältnis von Tyrosin zu Phenylalanin beträgt 1:1 bis 1:3, bevorzugt 1:1,3 bis 1:2,5 und besonders bevorzugt 1:1,5 bis 1:2.

Aufgrund der geringen Aktivität der Enzyme Cysteinsulfinsäuredecarboxylase und Cystathionase ist der Transsulfurationsmetabolismus bei Früh-, Neugeboren und Kleinkindern kaum ausgeprägt. Daher wird aus Methionin nicht oder nur mit sehr geringen Geschwindigkeiten Cystein und aus Cystein Taurin synthetisiert. Sowohl Cystein als auch Taurin sind daher für den neonatalen Organismus als essentiell anzusehen. Insbesondere Taurin spielt bei der Entwicklung von Früh- und Neugeborenen sowie Kleinkindern aber eine bedeutende Rolle für die Ausprägung des zentralen Nervensystems, des Verdauungssystems, des Seh- und Hörvermögens sowie des Calciumhaushalts in Zellen im Allgemeinen und Nervenzellen im Speziellen. Neben der Funktion als Proteinbaustein fungiert Cystein ebenfalls als Vorläufer des Tripeptids Glutathion, das eine wichtige Rolle bei der Verhinderung von oxidativem Stress spielt. Es hat sich gezeigt, dass auch im Vergleich zu bisher üblichen Dosen hohe Mengen an Taurin vom neonatalen Organismus gut akzeptiert werden.

Aus diesem Grunde sind in der Komposition die relativen Mengen an Cystein und Taurin erhöht, wohingegen die Menge an Methionin verringert ist. Die Gesamtmenge an Cystein, Taurin und Methionin überschreitet 5g / 100 g AA nicht, beträgt aber mindestens 2g / 100 g AA.

Die Komposition enthält 0,3-1,5g Cystein / 100 g AA, bevorzugt 0,5-1 g / 100 g AA. Die Komposition enthält weiterhin 0,3-2 g Taurin / 100 g AA, bevorzugt 0,7-1,5 g / 100 g AA.

Da Cystein als freie Aminosäure in wässrigen Lösungen nicht stabil ist und insbesondere keine Hitzesterilisation erlaubt, wird Cystein bevorzugt in Form des Precursors N-Acetyl-L-Cystein eingesetzt. Die Verabreichung in Form von anderen Vorstufen, insbesondere in Form von Oligopeptiden, ist im Rahmen der Erfindung ebenfalls möglich.

Die Herstellung von Aminosäurenlösungen enthaltend Glutamin und Tyrosin in adäquaten Mengen war ohne die Verwendung der stabilen und gut löslichen Oligopeptiden nicht möglich. Beide freien Aminosäuren besitzen eine geringe Löslichkeit. Zusätzlich erlaubt der Einsatz von freiem Glutamin keine Hitzesterilisation. Aus diesem Grunde wurde in der Vergangenheit oft Glutaminsäure als Ersatz für Glutamin eingesetzt. Glutaminsäure und Glutamin stehen im Körper in einem metabolischen Gleichgewicht, können aber nur zu einem gewissen Anteil körpereigen ineinander konvertiert werden. Deshalb kann Glutaminsäure nicht als vollwertiger Ersatz für Glutamin angesehen werden. Es wurde festgestellt, dass die Verabreichung hoher Mengen Glutaminsäure exzitatorisch wirkt und eine Überaktivität bei der Anregung von Nervenzellen bewirkt, die zum Zelltod führen kann [Barinaga (1990) Science 247:20-22]. Im Gegensatz dazu konnte festgestellt werden, dass eine Verabreichung erhöhter Mengen Glutamin unschädlich ist. Darüber hinaus lässt sich der Bedarf der Tagesdosis von Glutaminsäure durchaus über eine erhöhte Zufuhr von Glutamin abdecken. Damit ist eine angemessene parenterale Ernährung auch bei einer völligen Abwesenheit von Glutaminsäure möglich, wenn durch entsprechend hohe Dosen an Glutamin die Grundversorgung gewährleistet ist. Aus diesem Grunde übersteigen die anteiligen Mengen der erfinderischen Komposition an Glutamin auch deutlich bisher verwendete Mengen.

Die Lösung des Stabilitäts- und Löslichkeitsproblems besteht in der Bereitstellung von Glutamin in Form eines Dipeptids und zwar in Form von Alanyl-Glutamin, Glycyl- Glutamin oder Mischungen davon..

Die freie Aminosäure Tyrosin besitzt ebenfalls den Nachteil der geringen Löslichkeit. Die geringe Löslichkeit von Tyrosin hat in der Vergangenheit dazu geführt, dass Aminosäurelösungen unter Beibehaltung des gleichen Aminosäuremusters und Verwendung von freiem Tyrosin in einer geringeren Gesamtkonzentration als der idealen von etwa 10 % w/w Proteinbausteine (7-15 % w/w Proteinbausteine) hergestellt werden mussten. Daher wurde der Tyrosinanteil in Aminosäurelösungen zur parenteralen Ernährung bisher oft durch das besser lösliche Acetyl-Tyrosin gedeckt. Acetyl-Tyrosin wird allerdings vom menschlichen Körper schlecht metabolisiert [Magnusson et al. (1989) Metabolism 38:957-961], dies gilt in besonderem Maße für den neonatalen Organismus.

Eine Problemlösung besteht wiederum in der Bereitstellung von Tyrosin in Form eines Dipeptids, und zwar in Form von Glycyl-Tyrosin, Alanyl-Tyrosin oder Mischungen davon.

Die Bereitstellung von Glutamin und Tyrosin in Form der rasch metabolisierbaren genannten Dipeptide erlaubt die Herstellung einer hitzesterilisierten kompletten Aminosäurelösung bei gleichzeitig hoher Stabilität und Lagerfähigkeit.

Es hat sich überraschend herausgestellt, dass eine Hitzesterilisation bei höheren Sterilisationstemperaturen als üblich (>121°C) bei gleicher Sterilisationsleistung (definiert durch einen konstanten F0-Wert) unter größerer Stabilität der Dipeptide möglich ist. Die Sterilisationstemperatur beträgt dabei ≥124°C und besonders bevorzugt ≥127°C. Der pH-Wert ist bevorzugt zwischen 5 und 6,5 angesiedelt. Bei der Verwendung von höheren Temperaturen ist eine Verringerung der Haltezeiten der Hitzesterilisation möglich. Unter den bevorzugten Bedingungen werden in dem Sterilisationsprodukt deutlich geringere Mengen an Abbauprodukten gefunden (Beispiel 3).

Bei der totalen parenteralen Ernährung kann die erfindungsgemäße Aminosäurelösung vor der Verabreichung mit anderen parenteralen Nahrungskomponenten zu einer Komposition gemischt werden, die alle Erfordernisse einer vollständigen Ernährung erfüllt. Aufgrund der Wachstumsphase bei Früh- und Neugeborenen sowie Kleinkindern ist im Vergleich zu Erwachsenen der Kalorienbedarf in Bezug auf das Körpergewicht erhöht. Der neonatale Organismus benötigt eine Tagesdosis von 80-190 kcal/kg, bevorzugt 90-120 kcal/kg, für Kleinkinder nach dem ersten Lebensjahr fällt dieser Bereich auf 75-90 kcal/kg und unter diesen Bereich für ältere Kinder. Dabei ist es vorzuziehen, dass die in der parenteralen Ernährungstherapie zur Verfügung gestellten Aminosäuren ausschließlich für den Proteinaufbau und notwendige Stoffwechsel-Prozesse vorgesehen sind und nicht der allgemeinen Energiezufuhr dienen. Eine vollständige parenterale Ernährung stellt daher nur etwa 10-20% der Energie als Proteinbausteine zur Verfügung, 30-60 % werden durch Kohlehydrate, bevorzugt Glucose, und weitere 30-50 % durch Fette bereitgestellt. Elektrolyte, Vitamine und Spurenelemente komplettieren die parenterale Nahrungskomposition.

Es ist bekannt, dass Aminosäuren nicht gemeinsam mit Fetten und Kohlehydraten gelagert werden können wegen auftretender Stabilitätsprobleme. Daher werden diese Komponenten der parenteralen Ernährung erst kurz vor der Verabreichung unter sterilen Bedingungen zusammengemischt. Ein solches Mischen vor Verabreichung ist daher besonders zeitintensiv. Das Problem wurde durch Mehrkammerbeutel mit peelbaren Schweißnähten gelöst, wie z.B. in der EP 1396249 dargestellt. Die Verwendung eines solchen System bietet sich auch für die Verabreichung von Neoven gemäß der vorliegenden Erfindung an.

Die Erfindung soll anhand folgender Beispiele näher erläutert werden.

### Beispiel 1:

In der nachfolgenden Tabelle werden auf Basis eines Volumens von 1000 ml die Eigenschaften der erfindungsgemäßen Aminosäurelösung aufgezeigt. Es werden mögliche Mengenbereiche, bevorzugte Mengenbereiche, bevorzugte Tagesdosis sowie ein konkretes Beispiel der Mengen gegeben.

| 1000 ml Infusionslösung: | mögliche Mengenbereiche | bevorzugte Mengenbereiche | bevorzugte Tagesdosis pro kg | Beispiel |
|---|---|---|---|---|
| L-Leucin | 5-20 g | 9-12 g | 150-610 mg | **10.8 g** |
| L-Isoleucin | 3-8 g | 4-6 g | 90-250 mg | **4.8 g** |
| L-Lysin | 5-15 g | 7-10 g | 150-450 mg | **8.6 g** |
| L-Methionin | 1-3 g | 1,5-2,5 g | 30-90 mg | **2.0 g** |
| L-Phenylalanin | 3-5 g | 3,3-4 g | 90-150 mg | **3.6 g** |
| L-Threonin | 3-8 g | 5-6 g | 90-250 mg | **5.5 g** |
| L-Tryptophan | 1-4 g | 1,5-2,8 g | 30-120 mg | **2.2 g** |
| L-Valin | 3-8 g | 5-6 g | 90-250 mg | **5.5 g** |
| L-Arginin | 3-12 g | 6-9 g | 90-370 mg | **8.0 g** |
| L-Histidin | 1,5-6 g | 2-4 g | 45-180 mg | **3.2 g** |
| L-Alanin | 5-15 g | 8-12 g | 150-450 mg | **9.7 g** |
| Glycin | 1,5-6 g | 2,5-4 g | 45-180 mg | **3.2 g** |
| Glycyl-L-Tyrosin | 0,7-5 g | 2-3,3 g | 20-150 mg | **2**.**7g** |
| L-Tyrosin | 1-4 g | 1,5-2,5 g | 30-120 mg | **2.0 g*** |
| L-Prolin | 5-15 g | 7-10 g | 150-450 mg | **8.6 g** |
| L-Serin | 3-8 g | 4-6 g | 90-250 mg | **5.8 g** |
| L-Cystein(acetyl) | 0,3-1,5 g | 0,5-1 g | 9-45 mg | **0.7 g (1.0 g)** |
| L-Alanyl-L-Glutamin | 10-44 g | 17-30 g | 0,3-1,3 g | **22.0g** |
| L-Glutamin | 9-30 g | 11-20 g | 250-900 mg | **15.0 g **** |
| L-Glutaminsäure | 0-10 g | 0-3 g | 0-300 mg | **0** |
| Taurin | 0.3-2 g | 0,7-1,5 g | 9-60 mg | **1.0 g** |
| AA g | 30-200 g | 80-150 g | 2-4 g | **100.5 g** |
| EAA | 15-90 g | 30-60 g | 0,4-2,8 g | **43.0 g** |
| BCAA | 7-40 g | 15-30 g | 0,2-1,2 g | **21**.**1g** |
| NEAA | 20-120 g | 45-80 g | 0,6-3,6 g | **57.5 g** |
| N (Stickstoff) | 4,8-32 | 12,8-24 | 0,32-0,64 | **16**.**0 g** |
| pH | 5-7 | 5-6,5 | | **5.8 -6.2** |
| Titrationsacidität [mmol NaOH] | < 40 | <35 | | **30** |
| Osmolarität [mosmol/l] | 300-1000 | 700-900 | | **785** |

| | | | | |
|---|---|---|---|---|
| ** in Form von L-Alanyl-L-Glutamin * in Form von Glycyl-L-Tyrosin AA = Aminosäuren EAA = essentielle Aminosäuren BCAA = Aminosäuren mit verzweigter Kette (branched chain) | | | | |

### Beispiel 2:

Eine Lösung zur vollständigen parenteralen Ernährung eines pädiatrischen Patienten enthält pro kg Körpergewicht für die Infusion einer Tagesdosis über 24 Stunden:

| | |
|---|---|
| 12-35 ml | Aminosäurelösung nach Beispiel 1 |
| 20-120 ml | Glukoselösung 5-20 % |
| 2-15 ml | Lipidlösung |
| 2-3 mmol | Natrium |
| 2-3mmol | Kalium |
| 0,1-0,6 mmol | Kalzium |
| 0,1-0,8 mmol | Magnesium |
| 0,5-15 µg | Vitamin D |
| 2-7 mg | Vitamin E |
| 10-200 µg | Vitamin K |
| 10-80 mg | Vitamin C |
| 0,3-1,5 mg | Vitamin B1 |
| 0,1-1,5 mg | Vitamin B2 |
| 0,1-1,5 mg | Vitamin B6 |
| 0,2-1,2 µg | Vitamin B12 |
| 4-18 mg | Niacin |
| 50-150 µg | Folsäure |
| 50-500 µg | Zink |
| 2-3 µg | Selen |
| 30-200 µg | Eisen |

### Beispiel 3:

Eine Lösung von 200 g/l Alanyl-Glutamin wurde bei Temperaturen von 121° C, 124°C und 127°C sterilisiert. Der pH-Wert wurde zwischen pH 5, pH 5,5 und pH 6 variiert. Die Haltezeiten der Hitzesterilisation wurden so variiert, dass vergleichbare F0-Werte zwischen 12 und 12,5 erreicht wurden, die belegen, dass die Sterilisationsleistung auf gleichem Niveau bleibt. Der F0-Wert beschreibt die Sterilisationsleistung und entspricht der Haltezeit bei 121,11 °C. Bei einer Sterilisation mit höheren Temperaturen wurden die Haltezeiten verringert unter Beibehaltung eines konstanten F0-Wertes. Nach der Sterilisation wurden die Mengen der Abbauprodukte des Dipeptids Alanyl-Glutamin, cyclo-Alanyl-Glutamin und L-pyro-Alanyl-Glutamin, bestimmt. Niedrige Werte für diese Abbauprodukte sind ein direktes Indiz für die Stabilität des Dipeptids während der Sterilisation. Die Werte für die Abbauprodukte sanken bei höherer Temperatur.

Die Ergebnisse der Sterilisationsversuche sind in nachfolgender Tabelle dargestellt

| **pH-Wert** | **Tₛₜ** | **c-ala-gln [%]** | **pyro-glu-ala [%]** | **F0 Wert** |
|---|---|---|---|---|
| 5,0 | 121°C | 1,09 | 0,36 | 12,1 |
| 5,5 | 121°C | 1,31 | 0,30 | 12,1 |
| 6,0 | 121°C | 1,54 | 0,28 | 12,1 |
| 5,0 | 124°C | 0,89 | 0,30 | 12,5 |
| 5,5 | 124°C | 1,08 | 0,26 | 12,5 |
| 6,0 | 124°C | 1,25 | 0,24 | 12,5 |
| 5,0 | 127°C | 0,76 | 0,26 | 12,5 |
| 5,5 | 127°C | 0,9 | 0,22 | 12,5 |
| 6,0 | 127°C | 1,08 | 0,21 | 12,5 |

### Beispiel 4:

Eine Lösung zur parenteralen Verabreichung wird in einem Zweikammerbeutel mit peelbarer Trennwand bereitgestellt. Eine Kammer des Beutels enthält die erfindungsgemäße Aminosäurelösung, die andere Kammer enthält eine Ernährunglösung enhaltend Glukose, Elektrolyte, Vitamine und Spurenelemente. Die Zusammensetzung eignet sich besonders für pädiatrische Patienten im Alter von einem Monat bis ein Jahr.

| **Zusammensetzung** | Bevorzugter Bereich | Konkretes Beispiel |
|---|---|---|
| Volumen ml | 100 | 100,0 |
| AA g (nach Bsp. 1) | 2-3 | 2,4 |
| Glukose g | 9-15 | 12,0 |
| Na mol | 2-3 | 2,40 |
| K mmol | 1,5-2,5 | 2,00 |
| Ca mmol | 1-1,8 | 1,44 |
| Mg mmol | 0,1-0,3 | 0,20 |
| P mmol | 0,9-1,4 | 1,12 |

Beutel mit entsprechenden Zusammensetzungen werden in den Größen 350 ml, 500 ml und 1000 ml angeboten um Patienten unterschiedlicher Altersgruppen mit einer angemessenen Menge zu versorgen.

### Beispiel 5:

Eine Lösung zur parenteralen Verabreichung wird in einem Dreikammerbeutel mit peelbarer Trennwand bereitgestellt. Eine Kammer des Beutels enthält die erfindungsgemäße Aminosäurelösung, eine Kammer enthält eine Ernährunglösung enhaltend Glukose, Elektrolyte, Vitamine und Spurenelemente. Eine weitere Kammer enthält eine Fettemulsion. Die Fettemulsion enthält bevorzugt Fette aus Sojabohnenöl, mittelkettigen Fettsäuren (MCT), Olivenöl und Fischöl. Die Zusammensetzung eignet sich besonders für pädiatrische Patienten mit einem Gewicht von 10 - 40 kg und einem Alter von mehr als einem Jahr. Generell kann aber auch von einer Eignung für jüngere Patienten ausgegangen werden.

| **Zusammensetzung** | Bevorzugter Bereich | Konkretes Beispiel |
|---|---|---|
| Volumen ml | 100 | 100 |
| Gesamtkalorien | 60-100 | 80 |
| AA g (nach Bsp. 1) | 1,5-2,5 | 2 |
| Glukose g | 9-15 | 12 |
| Fette g (SMOFlipid) | 1,5-2,5 | 2 |
| Na mmol | 2-2,5 | 2,22 |
| K mmol | 2-2,5 | 2,22 |
| Ca mmol | 0,4-0,7 | 0,56 |
| Mg mmol | 0,05-0,15 | 0,11 |
| P mmol | 0,6-1 | 0,78 |

Beutel mit entsprechenden Zusammensetzungen werden in den Größen 1000 ml, und 2000 ml angeboten, um Patienten unterschiedlicher Gewichtsklassen mit einer angemessenen Menge zu versorgen.

## Patentansprüche

1. Aminosäurelösung zur parenteralen Ernährung pädiatrischer Patienten, **dadurch gekennzeichnet, dass** die Aminosäurelösung 9 - 30 g Glutamin auf 100 g Aminosäuren, 0,3 - 2 g Taurin auf 100 g Aminosäuren,
1 - 4 g Tyrosin auf 100 g Aminosäuren, 0,3 - 1 ,5 g Cystein auf 100 g Aminosäuren, 3 - 5 g Phenylalanin auf 100 g Aminosäuren und 1 - 3 g Methionin auf 100 g Aminosäuren enthält; wobei Glutamin in Form von Alanyl-Glutamin, Glycyl- Glutamin oder Mischungen davon vorliegt und wobei Tyrosin in Form von Glycyl-Tyrosin, Alanyl-Tyrosin oder Mischungen davon vorliegt.

2. Aminosäurelösung nach Anspruch 1, **dadurch gekennzeichnet, dass** die Aminosäurelösung höchstens 3 g/l Glutaminsäure enthält.

3. Aminosäurelösung nach einem der vorhergehenden Ansprüche, **dadurch gekennzeichnet, dass** die Aminosäurelösung 3 - 30 % w/w Proteinbausteine enthält.

4. Aminosäurelösung nach einem der Ansprüche 1 oder 2, **dadurch gekennzeichnet, dass** das Verhältnis von Tyrosin zu Phenylalanin 1 :1 bis 1 :3 beträgt.

5. Aminosäurelösung nach einem der Ansprüche 1 bis 4, **dadurch gekennzeichnet, dass** der gemeinsame Gewichtsanteil der Aminosäuren Cystein, Taurin und Methionin 2 - 5 % aller Aminosäuren ausmacht.

6. Aminosäurelösung nach einem der vorhergehenden Ansprüche, **dadurch gekennzeichnet, dass** die Aminosäurelösung weniger als 1 % Proteine oder Peptide mit einer Kettenlänge >5 Aminosäure-Bausteinen enthält.

7. Aminosäurelösung nach Anspruch 1 zur Verwendung für die parenterale Ernährung eines pädiatrischen Patienten.

8. Aminosäurelösung nach Anspruch 7, **dadurch gekennzeichnet, dass** der Patient an einer Missbildung oder an einer entzündlichen Darmerkrankung leidet.

9. Aminosäurelösung nach Anspruch 1 zur Verwendung für die parenterale Ernährung eines Patienten mit einer Nieren- oder Lebererkrankung.

10. Medizinische Zusammensetzung zur vollständigen parenteralen Ernährung, **dadurch gekennzeichnet, dass** sie a) 30 - 60 % der Energie durch Kohlehydrate, b) 30-50 % der Energie durch Fette, c) 10 - 20 % der Energie durch eine Aminosäurelösung gemäß Anspruch 1 bereitstellt.

11. Medizinische Zusammensetzung nach Anspruch 10, **dadurch gekennzeichnet, dass** die Nahrungsmittelzusammensetzung zusätzlich Elektrolyte, Vitamine und Spurenelemente enthält.

12. Medizinische Zusammensetzung nach Anspruch 10 in Form einer parenteralen Infusionslösung zur Behandlung pädiatrischer Patienten, **dadurch gekennzeichnet, dass** die Infusionslösung eine Tagesdosis von 90 - 180 kcal pro kg Körpergewicht des Patienten enthält.

13. Herstellungsverfahren einer sterilen Aminosäurelösung zur parenteralen Ernährung nach einem der Ansprüche 1-6 enthaltend 9 - 30 g Glutamin auf 100 g Aminosäuren in Form von Oligopeptiden, **dadurch gekennzeichnet, dass** es zumindest den Schritt der Hitzesterilisation bei einer Temperatur von ≥ 124 ⁰C enthält.

## Claims

1. Amino acid solution for the parenteral nutrition of paediatric patients, **characterized in that** the amino acid solution contains 9 - 30 g of glutamine to 100 g of amino acids, 0.3 - 2 g of taurine to 100 g of amino acids,
1 - 4 g of tyrosine to 100 g of amino acids, 0.3 - 1.5 g of cysteine to 100 g of amino acids, 3 - 5 g of phenylalanine to 100 g of amino acids and 1 - 3 g of methionine to 100 g of amino acids; glutamine being present in the form of alanylglutamine, glycyl-glutamine or mixtures thereof and tyrosine being present in the form of glycyl-tyrosine, alanyltyrosine or mixtures thereof.

2. Amino acid solution according to Claim 1, **characterized in that** the amino acid solution contains at most 3 g/l of glutamic acid.

3. Amino acid solution according to one of the preceding claims, **characterized in that** the amino acid solution contains 3 - 30% w/w of protein building blocks.

4. Amino acid solution according to one of Claims 1 or 2, **characterized in that** the ratio of tyrosine to phenylalanine is 1:1 to 1:3.

5. Amino acid solution according to one of Claims 1 to 4, **characterized in that** the joint proportion by weight of the amino acids cysteine, taurine and methionine makes up 2 - 5% of all amino acids.

6. Amino acid solution according to one of the preceding claims, **characterized in that** the amino acid solution contains less than the 1% of proteins or peptides with a chain length > 5 amino acid building blocks.

7. Amino acid solution according to Claim 1 for use for the parenteral nutrition of a paediatric patient.

8. Amino acid solution according to Claim 7, **characterized in that** the patient suffers from a malformation or from an inflammatory bowel disease.

9. Amino acid solution according to Claim 1 for use for the parenteral nutrition of a patient with a kidney or liver disease.

10. Medicinal composition for complete parenteral nutrition, **characterized in that** it provides a) 30-60% of the energy by carbohydrates, b) 30-50% of the energy by fats, c) 10-20% of the energy by an amino acid solution according to Claim 1.

11. Medicinal composition according to Claim 10, **characterized in that** the nutritional composition additionally contains electrolytes, vitamins and trace elements.

12. Medicinal composition according to claim 10 in the form of a parenteral infusion solution for the treatment of paediatric patients, **characterized in that** the infusion solution contains a daily dose of 90 - 180 kcal per kg of body weight of the patient.

13. Production process of a sterile amino acid solution for parenteral nutrition according to one of Claims 1-6 containing 9 - 30 g of glutamine to 100 g of amino acids in the form of oligopeptides, **characterized in that** it contains at least the step of heat sterilization at a temperature of ≥ 124°C.

## Revendications

1. Solution d'acides aminés pour alimentation parentérale de patients pédiatriques, **caractérisée en ce que** la solution d'acides aminés contient 9-30 g de glutamine pour 100 g d'acides aminés, 0,3-2 g de taurine pour 100 g d'acides aminés, 1-4 g de tyrosine pour 100 g d'acides aminés, 0,3-1,5 g de cystéine pour 100 g d'acides aminés, 3-5 g de phénylalanine pour 100 g d'acides aminés et 1-3 g de méthionine pour 100 g d'acides aminés ; la glutamine se présentant sous forme d'alanyl-glutamine, de glycyl-glutamine ou de mélanges de celles-ci, et la tyrosine se présentant sous forme de glycyl-tyrosine, d'alanyl-tyrosine ou de mélanges de celles-ci.

2. Solution d'acides aminés selon la revendication 1, **caractérisée en ce que** la solution d'acides aminés contient au plus 3 g/l d'acide glutamique.

3. Solution d'acides aminés selon l'une des revendications précédentes, **caractérisée en ce que** la solution d'acides aminés contient 3-30 % p/p de constituants protéiques.

4. Solution d'acides aminés selon l'une des revendications 1 ou 2, **caractérisée en ce que** le rapport de la tyrosine à la phénylalanine est de 1:1 à 1:3.

5. Solution d'acides aminés selon l'une des revendications 1 à 4, **caractérisée en ce que** la proportion pondérale des acides aminés cystéine, taurine et méthionine pris ensemble, est de 2-5 % de l'ensemble des acides aminés.

6. Solution d'acides aminés selon l'une des revendications précédentes, **caractérisée en ce que** la solution d'acides aminés contient moins de 1 % de protéines ou de peptides ayant une longueur de chaîne > 5 constituants acides aminés.

7. Solution d'acides aminés selon la revendication 1 pour utilisation pour l'alimentation parentérale d'un patient pédiatrique.

8. Solution d'acides aminés selon la revendication 7, **caractérisée en ce que** le patient souffre d'une malformation ou d'une affection intestinale inflammatoire.

9. Solution d'acides aminés selon la revendication 1, pour utilisation pour l'alimentation parentérale d'un patient présentant une maladie des reins ou du foie.

10. Composition médicinale pour alimentation parentérale complète, **caractérisée en ce qu'**elle fournit a) 30-60 % de l'énergie due aux glucides, b) 30-50 % de l'énergie due aux lipides, c) 10-20 % de l'énergie grâce à une solution d'acides aminés selon la revendication 1.

11. Composition médicinale selon la revendication 10, **caractérisée en ce que** la composition alimentaire contient en outre des électrolytes, des vitamines et des oligo-éléments.

12. Composition médicinale selon la revendication 10 sous forme d'une solution pour perfusion parentérale pour le traitement de patients pédiatriques, **caractérisée en ce que** la solution pour perfusion contient une dose journalière de 90-180 kcal par kg de poids corporel du patient.

13. Procédé de fabrication d'une solution stérile d'acides aminés pour alimentation parentérale selon l'une des revendications 1 à 6, contenant 9-30 g de glutamine pour 100 g d'acides aminés sous forme d'oligopeptides, **caractérisé en ce qu'**il comprend au moins l'étape de stérilisation à la chaleur à une température ≥ 124°C.
